# EUROPEAN PATENT APPLICATION

(11) **EP 3 808 253 A1**
(43) Date of publication of application: **21.04.2021**
(21) Application number: 19203141.7
(22) Date of filing: 15.10.2019
(51) Int. Cl.: A61B 5/00, A61B 5/024, A61B 5/1455, G06T 7/00

(54) **HIGH DYNAMIC RANGE VITAL SIGNS EXTRACTION**

(71) Applicant: Koninklijke Philips N.V., 5656 AG Eindhoven (NL)
(72) Inventor: WANG, Wenjing, 5656 AE Eindhoven (NL)
(74) Representative: Philips Intellectual Property & Standards

(57) **Abstract**

The present invention relates to a device (200), system (500) and method for determining a vital sign (150) of a subject (1) by using high dynamic range techniques to overcome the problems arising from a non-homogenous illumination of the subject (1). A camera (100) is preferably alternately switched between a mode of a high exposure time and a low exposure time to continuously record electromagnetic radiation (10) transmitted through or reflected from a skin area (4, 6) including one or more skin pixels of the subject (1). A plurality of detection signals (110), each representing a time signal derived from the electromagnetic radiation (10) are then further processed by various method steps, such as selecting high-quality skin-pixels from the different exposure states for signal extraction, interpolating and combining different exposure signals for vital signs extraction to increase the number of skin pixels for a more accurate vital sign extraction.

## Description

### FIELD OF THE INVENTION

The present invention relates to a device, system and method for determining a vital sign of a subject by using high dynamic range techniques.

### BACKGROUND OF THE INVENTION

Vital signs of a person, for example the heart rate (HR), the respiration rate (RR) or the arterial blood oxygen saturation, serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography (PPG). PPG generally refers to the measurement of volume changes of an organ or a body part and in particular to the detection of volume changes due to a cardio-vascular pulse wave traveling through the body of a subject with every heartbeat. PPG is an optical measurement technique that evaluates a time-variant change of light reflectance or transmission of an area or volume of interest and is based on the principle that blood absorbs light more than surrounding tissue. Hence, variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation can be determined.

Recently, non-contact remote PPG (rPPG) devices for unobtrusive measurements have been introduced. Remote PPG utilizes light sources or, in general radiation sources, disposed remotely from the subject of interest. Similarly, also a detector, e.g., a camera or a photo detector, can be disposed remotely from the subject of interest.

In practical applications of vital signs monitoring, dedicate, homogenous and diffuse light source is usually not possible, such as in fitness exercise, emergency department triage, driver monitoring in automotive (influenced by out-car ambient light changes). The non-homogenous light source creates different illuminations on a human face, also due to the 3D structure of the face. On a subject face, there might be both the shallows (dark clippings) and bright clippings, which renders vital signs extraction from these polluted pixels impossible.

US 10,052,038 B2 discloses a device and method for determining a vital sign of a subject, which aims at increasing the signal-to-noise ratio and the efficiency of artefacts caused by motion of the subject. This is solved according to one aspect by determining temporal and/or spatial properties of the recorded skin area by using adjacent frame differences and quality metric for determining the desired vital sign more accurately.

In spite of this and other promising approaches, there is still a need for a device, system or method that efficiently addresses the problems mentioned above arising from the non-homogenous illumination.

### SUMMARY OF THE INVENTION

It is an object of the present invention to provide a device, system and method that allow a more efficient and accurate vital sign extraction particularly in the case of non-homogenous illumination.

In a first aspect of the present invention, a device for determining a vital sign is presented that comprises a processing unit configured to:
obtain a plurality of detection signals, each representing a time signal derived from electromagnetic radiation transmitted through or reflected from a skin area including one or more skin pixels of the subject and detected by an optical sensor, whose exposure time is alternated between at least a first exposure time and a second exposure time during detection of the electromagnetic radiation,
select a first and second skin area and the corresponding first and second detection signal,
segment the first detection signal into a first signal trace including signal values of the first detection signal derived from electromagnetic radiation detected by the optical sensor with the first exposure time,
segment the second detection signal into a second signal trace including signal values of the second detection signal derived from electromagnetic radiation detected by the optical sensor with the second exposure time,
interpolate the first and second signal trace, and
determine a vital signal from the interpolated first and second signal traces.

In a further aspect of the present invention, a system for determining a vital sign of a subject is presented that comprises:
an optical sensor configured to detect electromagnetic radiation transmitted through or reflected from a skin area including one or more skin pixels of the subject and derive a plurality of detection signals, each representing a time signal derived from the detected electromagnetic radiation, wherein the exposure time of the optical sensor is alternated between at least a first exposure time and a second exposure time during detection of the electromagnetic radiation, and
the above-described device for determining a vital sign of a subject from the plurality of detection signals.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea to solve the problems arising from a non-homogenous illumination, i.e., the difficulties arising from extracting vital signs from a portion of low quality skin pixels with spatially different illuminations. These low quality skin pixels arise from both the dark and bright clippings, where the dark (less illuminated) skin pixels are dominated by sensor noise and the bright skin pixels are clipped and thus do not allow extracting a decent PPG signal. These low quality skin pixels have to be abandoned/rejected for vital signs extraction.

This problem is tackled according to the present invention by using multiple exposure times of a camera to create a high dynamic range (HDR) sensing for vital signs extraction. The multiple exposure times are at least two different exposure times. Though one can in general adjust/adapt the camera exposure time to improve the measurement (by using auto-exposure or quality metric), it still cannot solve the fundamental limitation that a face has spatially different illuminations, as the exposure time is the same for all pixels in one video frame.

By combining the results of different exposure times, the number of skin pixels that can be used for physiological signal extraction is (significantly) increased in HDR sensing. Different skin areas (without bright or dark clippings) at different exposure times are selected for signal extraction. The way to implement the invention is very specific and non-trivial, which includes the approach of changing exposure times sequentially, select high-quality skin-pixels from different exposure states for signal extraction, interpolate and combine different exposure signals for vital signs extraction.

The detection signals are derived from electromagnetic radiation transmitted through or reflected from a skin area and thus include pulsatile signals and non-pulsatile signals. Further, these detection signals are an intensity in dependence on time, which is generally referred as to a time signal.

In this context, it shall be noted that the term "vital sign" shall be understood broadly in the sense of "physiological parameter" including not only vital signs in the strict sense, but also other physiological parameters like SvO2 or SaO2. The processing unit may thus be configured to determine as vital sign an indicator or concentration of blood components or species, in particular SpO2, SvO2, HBO2, HBCO, METHB, Bilirubin, and wherein the pulsatile component reflects the cardiac cycle or the respiratory cycle.

The processing unit is configured to select a first and second skin area and the corresponding first and second detection signals. The second skin area might be the same as the first skin area, but in the most general case, these skin areas are different from each other. As the exposure time of the optical sensor is alternated between at least a first exposure time and a second exposure time, the first detection signal is only recorded for the first exposure time and the second detection signal is only recorded for the second exposure time. Thus, these detection signals are segmented into respective traces as the optical sensor is preferably continuously recording the plurality of detection signals. Thus, the first signal trace comprises gaps or missing values at times at which the optical sensor has been operating at the second exposure time and the second signal trace comprises gaps or missing values at times at which the optical sensor has been operating at the first exposure time.

In a next step, the processing unit is configured to interpolate said first and second signal trace. This allows filling the missing values of the respective traces to obtain interpolated signal traces. Based on these interpolated signal traces it is then possible to determine accurate vital signs.

According to an embodiment, the processing unit is configured to generate a control signal to control the optical sensor to alternately switch between the first and second exposure time. In this context, it shall be understood that the term "alternately" means that the optical sensor switches at least once between different exposure times. However, in a preferred embodiment the optical sensor switches various times between different exposure times sequentially.

If the processing unit is configured to control the optical sensor to switch between said exposure times, a compact device for determining a vital sign is presented. Alternatively, an external control unit may be connected to the optical sensor and may be configured to generate a corresponding control signal to control the optical sensor to switch between the different exposure times. Then, the external control unit is preferably also connected to the device for determining a vital sign to send the device messages to transmit the device information about the timing of the different exposure times. Alternatively, the processing unit is configured to readout the different exposure times from the optical sensor.

According to a preferred embodiment, the processing unit is configured to generate the control signal to control the optical sensor to alternately switch between the first and second exposure time at a switching rate that is larger than a sampling rate of the optical sensor. A larger sampling rate allows denser exposure time changes and even more different exposure states (e.g., five different exposure times instead of only two). If the rate for changing the exposure time is smaller than the sampling rate of the optical sensor, it can lead to situations in which the optical sensor records images with a mixture of different exposure times. This is not desirable and complicates the past-processing of the detection signals.

It shall be understood that the present invention is not limited to the case of two different exposure times and two (different) skin areas. According to another embodiment, the processing unit is configured to select three or more skin areas and corresponding detection signals derived from electromagnetic radiation detected by the optical sensor, whose exposure time is alternated between three or more exposure times during detection of the electromagnetic radiation, segment the three or more detections signal into three or more signal traces, interpolate the three or more signal traces, and determine a vital signal from the interpolated three or more signal traces. This is in particular of interest if the rate of the optical sensor of switching between different exposure times is larger than the sampling rate. Selecting more than two skin areas allows obtaining more than two vital signs and thus allows averaging more vital signs to obtain a more accurate final result, which is preferably even obtained from a larger skin area and thus more reliable.

According to another embodiment, the processing unit is configured to select two or more first skin areas and corresponding first detection signals derived from electromagnetic radiation detected by the optical sensor with the same exposure time, combine the selected first detection signals into a combined detection signal, and use the combined first detection signal for subsequent segmentation. Thus, the first skin area may be split into two or more first (sub-)skin areas and the corresponding combined first detection signal is thus a combination of various first (sub-)detection signals. This allows using the combined information of more skin areas for the same exposure time to obtain more accurate results in the subsequent segmentation.

According to another embodiment, the processing unit is configured to normalize and/or filter the interpolated signals traces and determine a vital signal from the normalized and/or filtered first and second signal traces. It shall be understood that "normalize" means in said context that the processing unit is configured to divide the pulsatile components (AC components) of the respective detection signals by the non-pulsatile components (DC components). Preferably, the processing unit is configured to first normalize the interpolated signal traces and then filter the normalized interpolated signal trace. However, the present invention is not limited to said order and it works vice versa.

Further, there are two options to determine a vital sign. The processing unit may be either configured to determine a vital sign by combining the interpolated signal traces and then determining a vital sign from the combined signal traces or by determining a first and second vital sign from the interpolated signal traces and then combining said first and second vital sign to obtain a final vital sign.

According to another embodiment, the processing unit is configured to select a skin area and the corresponding detection signal based on one or more of a brightness value, chrominance value and modulation value.

Different exposure times may lead to different bright and dark skin areas. Thus, for each exposure time a proper skin area has to be selected by the processing unit. This can be based, e.g., on the brightness. Then, the processing unit is configured to select a skin area that is bright and thus reflects a lot of electromagnetic radiation back to the optical sensor. Alternatively, the processing unit may also be configured to select the skin area based on a chrominance value or a modulation value. The latter option allows selecting skin area with lots of pulsatile vessels to be recorded.

Preferably, the processing unit is configured to select the skin area and the corresponding detection signal by comparing the detection signal per skin area to a threshold value, in particular for brightness, chrominance and/or modulation. A mixture of the different threshold values is also a viable option to select the most suitable skin area for further processing.

According to an embodiment, the processing unit is configured to interpolate the segmented signal traces by use of an interpolation method using nearest non-missing values or a linear, cubic, mean-averaging or median-averaging interpolation method.

The optical sensor is preferably a monochrome or multi-spectral camera or photodiode array operating in the visible and/or infrared spectral range as PPG is typically recorded in said spectral range. Further, the optical sensor comprises at least two pixels to have a spatial resolution. Preferably, the optical sensor comprises a plurality of pixels and is further configured to detect electromagnetic radiation from different overlapping skin areas so that the detection signals are derived from electromagnetic radiation from different overlapping skin areas.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an embodiment of a system for determining a vital sign of a subject according to the present invention;
Fig. 2 shows a flowchart illustrating a method to be executed by the device according to the present invention;
Fig. 3 shows a flowchart illustrating another optional method to be executed by the device according to the present invention;
Fig. 4 shows a flowchart illustrating another optional method to be executed by the device according to the present invention;
Fig. 5 shows a flowchart illustrating another optional method to be executed by the device according to the present invention; and
Fig. 6 shows schematic diagrams illustrating method steps to be executed by the device according to an embodiment of the present invention.

### DETAILED DESCRIPTION OF THE INVENTION

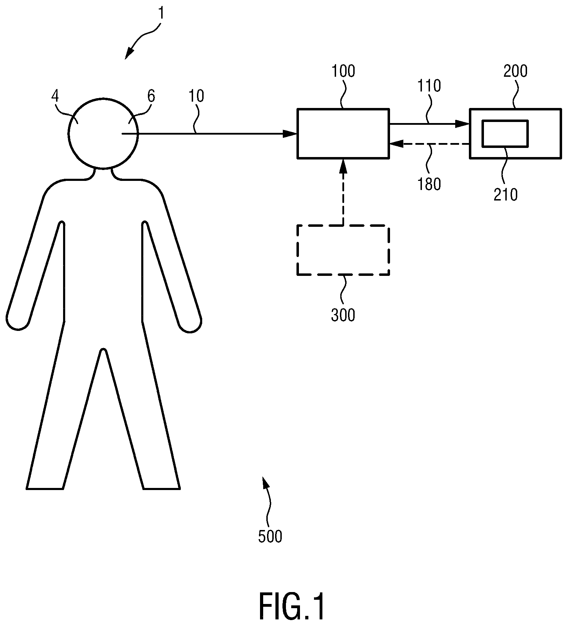

Fig. 1 shows a schematic diagram of an embodiment of a system 500 according to the present invention. The system 500 comprises an optical sensor 100 and a device 200 for determining a vital sign of a subject 1, wherein the device 200 further comprises a processing unit 210.

The optical sensor 100 is configured to detect electromagnetic radiation 10 transmitted through or reflected from a skin area 4, 6 of the subject 1. The skin area may be split into at least a first skin area 4 and a second skin area 6. Further, said optical sensor 100 is configured to derive a plurality of detection signals 110, wherein each of said detection signals 110 represents a time signal derived from the electromagnetic radiation 10. The optical sensor 100 preferably continuously records images during the measurement. The exposure time of the optical sensor 100 is alternated between a first exposure time and a second exposure time during the detection of electromagnetic radiation 10. Preferably, the electromagnetic radiation 10 is located in the red and infrared spectral range. For said purpose, the system 500 may further comprise a radiation source (not shown) configured to emit electromagnetic radiation in said spectral range onto the subject 1. The radiation source and the optical sensor 100 are arranged such that the electromagnetic radiation emitted by the radiation source and transmitted through or reflected from the skin 4, 6 of the subject 1 can be detected by the optical sensor 100. However, it should be noted that the present invention is particularly attractive for realistic applications where daylight is used and no specific radiation source is available.

The switching between the different exposure times of the optical sensor 100 may be caused by the processing unit 210. According to an embodiment, the processing unit 210 is configured to generate a control signal 180 to control the optical sensor 100 to alternately switch between the first and second exposure time. Alternatively, the system 500 may also comprise an external control unit 300 configured to generate such a control signal to control the optical sensor 100 to switch between the different exposure states.

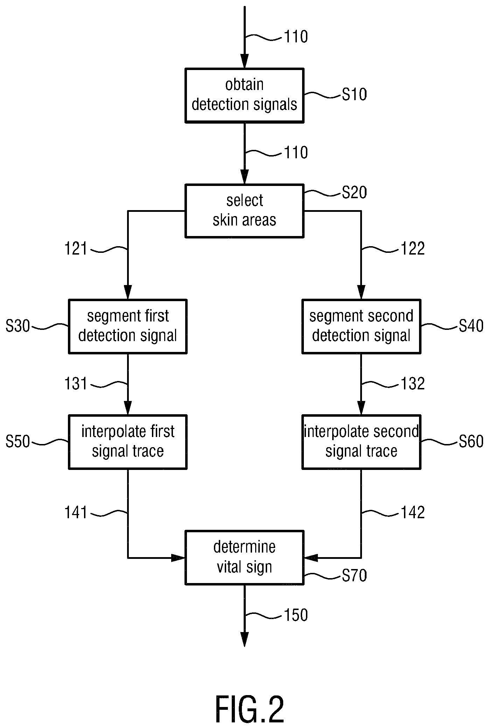

Fig. 2 shows a flowchart illustrating a method to be executed by the processing unit 210 of the device 200 for determining a vital sign 150 of a subject according to the present invention.

In a first step S10, the plurality of detection signals 110 mentioned above are obtained.

In a next step 20, a first skin area 4 and a second skin area 6 and the corresponding first and second detection signal 121, 122 are selected.

This first and second detection signal 121, 122 are then in the next steps S30, S40 segmented into a first signal trace 131 and into a second signal trace 132. The first signal trace 131 includes signal values of the first detection signal 121 derived from the electromagnetic radiation 10 detected by the optical sensor 100 with the first exposure time and the second signal trace 132 includes signal values of the second detection signal 122 derived from the electromagnetic radiation 10 detected by the optical sensor 100 with the second exposure time.

In further steps S50, S60, the first and second signal trace 131, 132 are interpolated and in a final step S70, a vital sign 150 is determined from the interpolated first and second signal trace 141, 142.

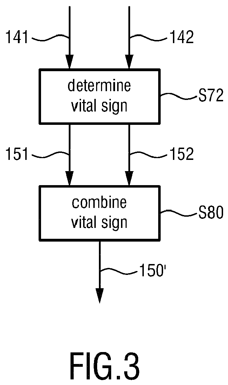

There are two options of how to determine a vital sign 150 in the last step S70. Either by first combining the interpolated signal traces 141, 142 and then determining a vital sign 150 from the combined signal traces (as shown in Fig. 2) or by first determining a first and second vital sign 151, 152 from the interpolated signal traces 141, 142 in a step S72 and then combining said first and second vital sign 151, 152 in a subsequent step S82 to obtain a final vital sign 150'. This second option is illustrated in the flowchart illustrated in Fig. 3.

Furthermore, it should be noted that two skin areas 4, 6 is only exemplarily and the processing unit 210 is configured to select even more skin areas 4, 6, and corresponding detection signals 121, 122, derived from the electromagnetic radiation 10 detected by the optical sensor 100. Thus, the optical sensor 100 may also switch between more than two different exposure times during detection of the electromagnetic radiation 10, wherein the switching rate, at which the exposure time is switched is, is preferably larger than the sampling rate of the optical sensor 100.

According to another embodiment, the order of the method steps illustrated in Fig. 2 may also be slightly different. It may be an option to execute the following method steps in the given order:
time sequential exposure settings,
obtain detection signals for the first exposure time,
select skin area(s) from the obtained detection signals with the first exposure time,
obtain detection signals for the second exposure time, and
select skin area(s) from the obtained detection signals with the second exposure time.

Thus, especially the step S10 of obtaining the detection signals may be preferably performed in a chronological sequence for the different exposure times.

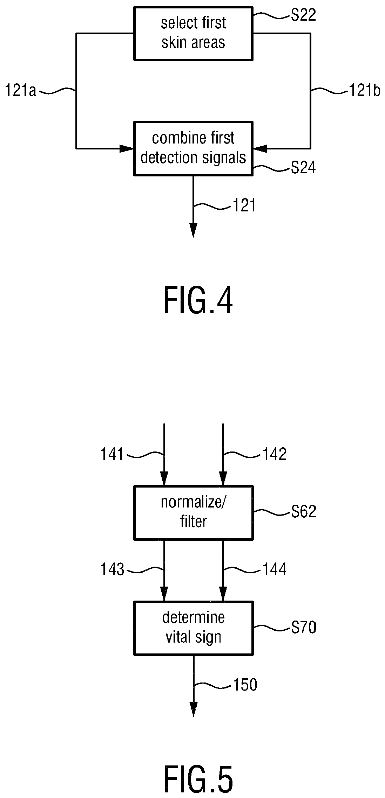

Fig. 4 shows a flowchart illustrating another optional method to be executed by the device 200 for determining a vital sign 150 of the subject 1 according to the present invention. In a step S22 , two or more first (sub-)skin areas 4a, 4b, ... and corresponding first (sub-)detection signals 121a, 121b, ... derived from electromagnetic radiation 10 detected by the optical sensor 100 with the same exposure time are selected. Thus, after the step S20 of selecting a first skin area 4 and a second skin area 6 (cf. Fig. 2) it may be a viable option to further process the detection signals derived from electromagnetic radiation reflected from or transmitted through sub-areas of the first skin area 4 detected by the optical sensor 100 at the first exposure time. In a next step S24, the first (sub-)detection signals 121a, 121b, corresponding to said first (sub-)skin areas 4a, 4b, are then combined to obtain the first detection signal 121 for the subsequent segmentation (as discussed above with reference to Fig. 2). This allows using the combined information of more discrete skin areas recorded for the same exposure time to obtain more accurate results in the subsequent segmentation.

Fig. 5 shows a flowchart illustrating another optional method to be executed by the device 200 for determining a vital sign 150 of a subject 1 according to the present invention. In a step S62, the interpolated signal traces 141, 142 are normalized and/or filtered and in a subsequent step S70, a vital sign 150 is determined from the normalized and/or filtered first and second signal traces 143, 144. Filtering the normalized signals allows further improving the signal-to-noise ratio and thus determining more accurate vital signs in the end.

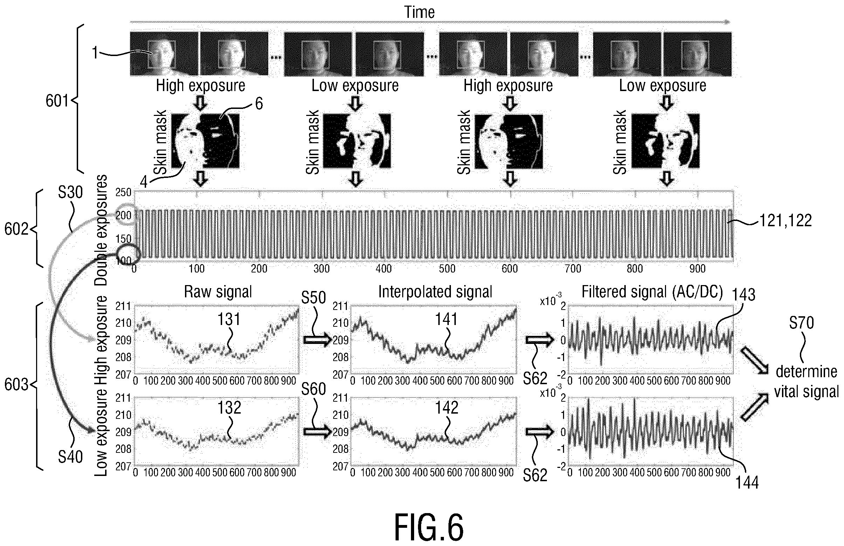

Fig. 6 shows schematic diagrams illustrating method steps to be executed by the processing unit 210 of the device 200 for determining a vital sign 150 of a subject 1 according to an embodiment of the present invention. It is illustrated in the first row 601 that the optical sensor 100 records a plurality of images of a skin region 4, 6 of the subject in a temporal sequence. This is preferably achieved by recording a video with a high and constant frame rate, e.g., 80 frames per second. The skin area 4, 6 is exemplarily a part of the face of the subject 1. The exposure time of the optical sensor is switched between a first exposure time (high exposure) and a second exposure time (low exposure). It can be seen that different skin areas 4, 6 are bright or dark for the different exposure times. It is exemplarily shown that the first skin area 4 (mainly located on the left part of the face) is bright if the exposure time is high and dark if the exposure time is low. Vice versa, the second skin area 6 (mainly located on the right part of the face) is dark if the exposure time is high and bright if the exposure time is low.

As explained already with reference to Fig. 2, a first and second skin area 4, 6 and the corresponding detection signals 121, 122 are selected (cf. S20 in Fig. 2). The skin areas 4, 6 are preferably selected based on one or more of a brightness value, chrominance value and modulation value. Thus, a skin mask may be defined based on one of these values (or any combination of them). According to the example shown in Fig. 6, the first skin area 4 is selected for the first exposure time and the second skin area 6 is selected for the second exposure time due to the brightness for the respective exposure times.

The first and second detection signal 121, 122 are each representing a time signal derived from the electromagnetic radiation 10 transmitted through or reflected from a skin area 4, 6 of the subject. The diagram in the second row 602 of Fig. 6 illustrates this first and second detection signal 121, 122 in dependence on the time. The time represents the recording time of the optical sensor 100. The optical sensor 100 has at least two pixels to have spatial resolution and to differentiate between the first skin area 4 and the second skin area 6.

As further explained already with reference to Fig. 2, in the next steps S30, S40, the first detection signal 121 is segmented into a first signal trace 131 and the second detection signal 122 is segmented into a second signal trace 132. This first and second signal trace 131, 132 are illustrated in the third row 603 in dependence on the time. It can be seen that the first signal trace 131 comprises gaps or missing values at times at which the optical sensor 100 has been operating at the second exposure time and the second signal trace 132 comprises gaps or missing values at times at which the optical sensor 100 has been operating at the first exposure time. Thus, in the next steps S50, S60, the first and second signal trace 131, 132 are respectively interpolated to obtain respective interpolated signal traces 141, 142. In a next step S62, these interpolated signal traces 141, 142 are respectively normalized and/or filtered and in a final step S70, a vital sign 150 is determined from the interpolated first and second signal trace 141, 142.

The main application of the present invention is camera based vital signs monitoring in challenging (ambient, non-dedicated, non-homogenous) illumination conditions, such as fitness exercise, driver monitoring in automotive, emergency department triage or hand-held monitoring in outdoor environment. It is also highly relevant for professional care in clinical applications or home-based monitoring as using the HDR measurement allows obtaining robust vital sign extraction for sleep monitoring, neonatal monitoring and other kinds of monitoring.

Further, it shall be understood that the present invention may also be realized as a patient monitor (not shown in the figures) comprising the device 200 for determining a vital sign 150 of a subject 1 as discussed above and a display for visualization of the time-dependent plots generated by said device 200. Said patient monitor may thus be especially configured to visualize all the plots and diagrams illustrated in Fig. 6.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (200) for determining a vital sign (150) of a subject (1), comprising a processing unit (210) configured to:
obtain a plurality of detection signals (110), each representing a time signal derived from electromagnetic radiation (10) transmitted through or reflected from a skin area (4, 6) including one or more skin pixels of the subject (1) and detected by an optical sensor (100), whose exposure time is alternated between at least a first exposure time and a second different exposure time during detection of the electromagnetic radiation (10),
select a first and second skin area (4, 6) and the corresponding first and second detection signal (121, 122),
segment the first detection signal (121) into a first signal trace (131) including signal values of the first detection signal (121) derived from electromagnetic radiation (10) detected by the optical sensor (100) with the first exposure time,
segment the second detection signal (122) into a second signal trace (132) including signal values of the second detection signal (122) derived from electromagnetic radiation (10) detected by the optical sensor (100) with the second exposure time,
interpolate the first and second signal trace (131, 132), and
determine a vital signal (150) from the interpolated first and second signal trace (141, 142).

2. Device (200) according to claim 1,
wherein the processing unit (210) is configured to generate a control signal (180) to control the optical sensor (100) to alternately switch between the first and second exposure time.

3. Device (100) according to claim 2,
wherein the processing unit (210) is configured to generate a control signal (180) to control the optical sensor (100) to alternately switch between the first and second exposure time at a switching rate that is larger than a sampling rate of the optical sensor (100).

4. Device (100) according to any one of the preceding claims,
wherein the processing unit (210) is configured to
select three or more skin areas (4, 6,) and corresponding detection signals (121, 122,) derived from electromagnetic radiation detected by the optical sensor (100), whose exposure time is alternated between three or more exposure times during detection of the electromagnetic radiation (10),
segment the three or more detections signal (121, 122,) into three or more signal traces (131, 132,
interpolate the three or more signal traces (131, 132,), and
determine a vital signal (150) from the interpolated three or more signal traces (131, 132,).

5. Device (200) according to any one of the preceding claims,
wherein the processing unit (210) is configured to
select two or more first skin areas (4a, 4b,) and corresponding first detection signals (121a, 121b, ...) derived from electromagnetic radiation (10) detected by the optical sensor (100) with the same exposure time,
combine the selected first detection signals (121a, 121 b,) into a combined first detection signal (121), and
use the combined first detection signal (121) for subsequent segmentation.

6. Device (200) according to any one of the preceding claims,
wherein the processing unit (210) is configured to
normalize and/or filter the interpolated signal traces (141, 142) and determine a vital signal (150) from the normalized and/or filtered first and second signal traces (143, 144).

7. Device (200) according to any one of the preceding claims,
wherein the processing unit (210) is configured to determine a vital sign (150) by
first combining the interpolated signal traces (141, 142) and then determining a vital sign (150) from the combined signal traces or
first determining a first and second vital sign (151, 152) from the interpolated signal traces (141, 142) and then combining said first and second vital sign (151, 152) to obtain a final vital sign (150').

8. Device (200) according to any one of the preceding claims,
wherein the processing unit (210) is configured to select a skin area (4, 6) and the corresponding detection signal (110) based on one or more of a brightness value, chrominance value and modulation value.

9. Device (200) according to any one of the preceding claims,
wherein the processing unit (110) is configured to select a skin area (4, 6) and the corresponding detection signal (110) by comparing the detection signal per skin area to a threshold value, in particular for brightness, chrominance and/or modulation.

10. Device (200) according to any one of the preceding claims,
wherein the processing unit (210) is configured to interpolate the segmented signal traces 131, 132) by use of an interpolation method using nearest non-missing values or a linear, cubic, mean-averaging or median-averaging interpolation method.

11. System (500) for determining a vital sign (150) of a subject (1), said system (150) comprising:
an optical sensor (100) configured to detect electromagnetic radiation (10) transmitted through or reflected from a skin area (4, 6) including one or more skin pixels of the subject (1) and derive a plurality of detection signals (110), each representing a time signal derived from the detected electromagnetic radiation (10), wherein the exposure time of the optical sensor (100) is alternated between at least a first exposure time and a second exposure time during detection of the electromagnetic radiation (10), and
a device (200) for determining a vital sign of a subject (1) as claimed in any one of the preceding claims from the plurality of detection signals (110).

12. System (500) according to claim 11,
wherein the optical sensor (100) is a monochrome or a multi-spectral camera or photodiode array operating in the visible and/or infrared spectral range.

13. System (500) according to claim 11 or 12,
wherein the optical sensor (100) is configured to detect electromagnetic radiation from different or overlapping skin areas so that the detection signals (110) are derived from electromagnetic radiation (10) from different or overlapping skin areas.

14. Method for determining a vital sign (150) of a subject (1), said method comprising the steps of:
obtaining (S10) a plurality of detection signals (110), each representing a time signal derived from electromagnetic radiation (10) transmitted through or reflected from a skin area (4, 6) including one or more skin pixels of the subject (1) and detected by an optical sensor (100), whose exposure time is alternated between at least a first exposure time and a second different exposure time during detection of the electromagnetic radiation,
selecting (S20) a first and second skin area (4, 6) and the corresponding first and second detection signal (121, 122),
segmenting (S30) the first detection signal (121) into a first signal trace (131) including signal values of the first detection signal (121) derived from electromagnetic radiation (10) detected by the optical sensor (100) with the first exposure time,
segmenting (S40) the second detection signal (122) into a second signal trace (132) including signal values of the second detection signal (122) derived from electromagnetic radiation (10) detected by the optical sensor (100) with the second exposure time,
interpolating (S50, S60) the first and second signal trace (131, 132), and
determining (S70) a vital signal (150) from the interpolated first and second signal trace (141, 142).

15. Computer program comprising program code means for causing a computer to execute the method as claimed in claim 14, when said computer program is executed on said computer.
